# EUROPEAN PATENT SPECIFICATION

(11) **EP 4 204 425 B1**
(45) Date of publication and mention of the grant of the patent: **30.04.2025**
(21) Application number: 21758933.2
(22) Date of filing: 02.08.2021
(51) Int. Cl.: C07F 7/18, C07C 253/34

(54) **METHOD FOR THE HYDROSILYLATION OF AN OLEFINIC NITRILE**
VERFAHREN ZUR HYDROSILYLIERUNG EINES OLEFINISCHEN NITRILS
PROCÉDÉ D'HYDROSILYLATION D'UN NITRILE OLÉFINIQUE

(30) Priority: 28.08.2020 US 202063071388 P
(43) Date of publication of application: 05.07.2023
(73) Proprietor: Dow Silicones Corporation, Midland, MI 48686-0994 (US); Dow Global Technologies LLC, Midland, MI 48674 (US)
(72) Inventor: BERGMAN, Evan, Midland, MI 48640 (US); HASAN, Towhid, Midland, MI 48640 (US); PAISLEY, Sean, Midland, MI 48674 (US); PANAH, Reza, Midland, MI 48640 (US); REISCH, Sean, Horseheads, NY 14845 (US); SIMON, Chris, Midland, MI 48640 (US)
(74) Representative: Murgitroyd & Company
(86) International application number: PCT/US2021/044116
(87) International publication number: WO 2022/046360

(56) References cited:
- US-A1- 2020 270 284
- ARMAREGO W L F ET AL: "Purifcation of Organic Chemicals - Aliphatic Compounds", 1 January 2009 (2009-01-01), XP009530547, ISBN: 978-1-85617-567-8, Retrieved from the Internet <URL:https://books.google.nl/books?id=PTXyS7Yj6zUC&pg=PA88&dq=purification+allyl+cyanide&hl=nl&source=gbs_toc_r&cad=3#v=onepage&q=purification%20allyl%20cyanide&f=false>

## Description

### CROSS REFERENCE TO RELATED APPLICATIONS

This application claims the benefit of U.S. Provisional Patent Application Serial No. 63/071,388 filed on 28 August 2020 under 35 U.S.C. §119 (e).

### TECHNICAL FIELD

This invention relates to an improved method for the hydrosilylation of an olefinic nitrile and a hydridosilane. More specifically, the invention relates to an improved method for preparing 3-cyanobutyl-methyldimethoxysilane by platinum catalyzed hydrosilylation of 2-methyl-3-butenenitrile and methyldimethoxysilane.

### BACKGROUND

Preparation of 3-cyanobutyl-methyldimethoxysilane from 2-methyl-3-butenenitrile (an olefinic nitrile, which is commercially available) and methyldimethoxysilane by platinum catalyzed hydrosilylation has been performed. Typical catalyst loading requires an amount sufficient to provide over 200 ppm of platinum to the reaction mixture (based on combined weights of the 2-methyl-3-butenenitrile and the methyldimethoxysilane) to achieve sufficient reactant conversion. Platinum catalysts are costly and increasingly scarce.

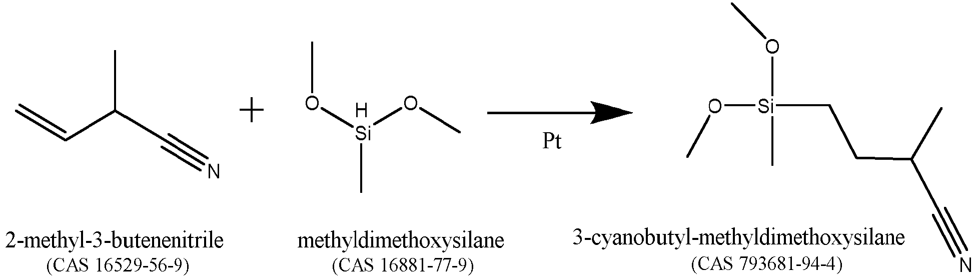

The following prior art may be useful in understanding the present invention. US2020270284A1 discloses a method for producing 4-(dialkylchlorosilyl)-butyronitriles which are prepared by high yield by reacting a mixture of technical grade allyl cyanide with dialkylchlorosilanes in the presence of a catalyst of transition group 8 of the Periodic Table of the Elements, wherein the reaction is conducted such that a stoichiometric excess of the dialkylchlorosilane is present together with the catalyst in the reaction mixture, into which the allyl cyanide is metered. ARMAREGO W L F ET AL, "Purifcation of Organic Chemicals - Aliphatic Compounds", ISBN 978-1-85617-567-8, (20090101), PURIFICATION OF LABORATORY CHEMICALS, ELSEVIER SCIENCE & TECHNOLOGY, PAGE(S) 98 discloses how to purify starting material allyl cyanide for example by distillation.

### BRIEF SUMMARY OF THE INVENTION

A method comprises:
1) providing A') an olefinic nitrile comprising a cyanide impurity of formula X'-C≡N, where X' is hydrogen, sodium, or potassium,
2) removing all or a portion of the cyanide impurity from A') the olefinic nitrile, thereby producing A) a purified olefinic nitrile; wherein step 2) comprises one or more of
   combining A') the olefinic nitrile and an elemental metal selected from the group consisting of copper, iron, silver, or nickel; or
   combining A') the olefinic nitrile and an adsorbent selected from the group consisting of activated carbon and molecular sieves; or
   combining A') the olefinic nitrile to a metal selected from the group consisting of copper, iron, silver, or nickel, or the oxides of these metal; and thereafter combining the product and an absorbent to form A) the purified olefinic nitrile; and
3) combining, at a temperature of 20 °C to 150 °C, starting materials comprising
   A) the purified olefinic nitrile;
   B) the hydridosilane, where the hydridosilane has formula R¹ₓHSiX₍₃₋ₓ₎, where subscript x is 0, 1, or 2; each R¹ is an independently selected monovalent hydrocarbon group of 1 to 18 carbon atoms, and each X is independently selected from the group consisting of a halogen atom and an alkoxy group of formula OR¹; and
   C) a platinum hydrosilylation reaction catalyst in an amount sufficient to provide 5 ppm to < 200 ppm Pt based on combined weights of starting materials A), B), and C).

### DETAILED DESCRIPTION OF THE INVENTION

Olefinic nitriles are known in the art and are commercially available. Suitable olefinic nitriles may be provided by commercial sources, such as Sigma-Aldrich of St. Louis, Missouri, USA; INVISTA North America S.ár.l; TCI America, Inc.; or TOKYO CHEMICAL INDUSTRY CO., LTD of Tokyo, Japan. The olefinic nitrile may have formula R-C≡N, where R represents an aliphatically unsaturated monovalent hydrocarbon group, such as an alkenyl group exemplified by vinyl, allyl, methyl propenyl, butenyl, methyl butenyl, and pentenyl. The olefinic nitrile is exemplified by acrylonitrile (CH₂=CHC≡N), 2-butenenitrile (CH₃-CH=CH-C≡N), 3-butenenitrile (CH₂=CH-CH₂-C≡N), 2-methyl-3-butenenitrile (shown above, CAS No. 16529-56-9), and 2-pentenenitrile (CH₃-CH₂-CH=CH-C≡N). Alternatively, the olefinic nitrile may be selected from the group consisting of 3-butenenitrile and 2-methyl-3-butenenitrile. Alternatively, the olefinic nitrile may be 2-methyl-3-butenenitrile.

The olefinic nitrile, as provided above, typically contains a cyanide impurity, where the cyanide impurity refers to a compound of formula X' -C≡N, where X' is hydrogen, sodium, or potassium. Alternatively, the cyanide impurity may be hydrogen cyanide of formula H-C≡N. The cyanide impurity may be present in an amount of 25 ppm or more, based on weight of the olefinic nitrile as provided. Alternatively, the cyanide impurity may be present in an amount of 30 ppm or more, alternatively 35 ppm or more, alternatively 40 ppm or more, alternatively 45 ppm or more, on the same basis. At the same time, the cyanide impurity may be present in an amount up to 200 ppm, alternatively up to 175 ppm, alternatively 150 ppm, and alternatively 125 ppm, on the same basis. Alternatively, the cyanide impurity may be present in an amount of > 25 ppm to 200 ppm, alternatively > 25 ppm to 125 ppm, and alternatively 45 to 125 ppm, based on weight of the olefinic nitrile as provided.

The inventors surprisingly found that cyanide impurities may inhibit hydrosilylation reaction of olefinic nitriles with hydridosilanes. Without wishing to be bound by theory, it is thought that H-C≡N content may be related to loss of catalyst activity as platinum catalyst loading decreased. Without wishing to be bound by theory, it is thought that removing all, or a portion, of the cyanide impurity from the olefinic nitrile before hydrosilylation of the olefinic nitrile and the hydridosilane can improve yield, enable reduced usage of expensive platinum catalyst, or both. Furthermore, process safety may also be improved when the hydrosilylation reaction proceeds more predictably, is less susceptible to pooling of reactants, and/or minimize SiH content of by-products.

In step 2) of the method described above, the purified olefinic nitrile may be prepared by removing all, or a portion, of the cyanide impurity. Methods for removing the cyanide impurity include contacting the olefinic nitrile with an adsorbent such as activated carbon or molecular sieves. The activated carbon may be wood, coconut, or lignite based; can be treated by various methods; and can be sized to provide adequate surface area for contacting. For example, one suitable type of activated carbon for use herein comprises acid-washed lignite-based activated carbon with a mesh size of 12x40 giving a mean particle size of 1 mm. Contacting the olefinic nitrile with the adsorbent in step 2) may be done under an inert atmosphere. Pressure for contacting may vary, e.g., from 0-345 kPa (0-50 psig). Temperature may vary, e.g., from 20-60 °C. Time for contacting may vary, e.g., for 1-24 hours, alternatively 1 to 8 hours. The amount of adsorbent may be 1% to 10%, alternatively 3% to 5%, based on combined weight of the adsorbent and the olefinic nitrile. Particle size of the adsorbent may be, for example, 1 mm to 2 mm.

Alternatively, step 2) may comprise contacting the olefinic nitrile with a metal absorber comprising a metal selected from the group consisting of iron (Fe), copper (Cu), silver (Ag), and nickel (Ni) as a powder, particle or metal on substrate. The metal absorber may be elemental Fe, Cu, Ag, or Ni. Alternatively, the metal absorber may be selected from the group consisting of elemental Fe, Cu, and Ag. Alternatively, the metal absorber may be a metal described above bound to a substrate such as carbon or silica. The metal absorber may be used under conditions to avoid or minimize oxidation of the metal, such as inert conditions. Contacting can be performed under a wide range of pressures, such as 0-345 kPa (0-50 psig). Contacting can be performed under a variety of temperatures, such as 20-60 °C being preferred. Contacting can be performed under a wide range of pressures, such as 0-345 kPa (0-50 psig). Contacting can be performed under a variety of temperatures, such as 20-60 °C. Contacting may be performed for a time e.g., for 1-24 hours, alternatively 1 to 8 hours. The amount of metal absorber may be 1% to 10%, alternatively 3% to 5%, based on combined weight of the metal absorber and the olefinic nitrile. Particle size of the metal absorber may be, for example, 1 mm to 2 mm. Without wishing to be bound by theory, it is thought that the metal may form a complex of the metal and cyanide impurity, which may then be removed by filtration and/or by contacting with an adsorbent, such as activated carbon, as described above. Without wishing to be bound by theory, it is thought that contacting the olefinic nitrile with the metal absorber may form a cyanide - metal complex, and the cyanide - metal complex may be then be removed by contacting with the adsorbent, as described above (e.g., when the metal is soluble) and/or by filtration (e.g., when a metal bound to a substrate is used, or after contacting with the adsorbent).

Alternatively, step 2) may comprise contacting the olefinic nitrile with a metal oxide absorber selected from the group consisting of oxides of Fe, Cu, Ag, or Ni. Contacting can be performed under a wide range of pressures, such as 0-345 kPa (0-50 psig). Contacting can be performed under a variety of temperatures, such as 20-60 °C being preferred. Contacting in step 2) may be done under an inert atmosphere. Contacting can be performed under a wide range of pressures, such as 0-345 kPa (0-50 psig). Contacting can be performed under a variety of temperatures, such as 20-60 °C. Contacting may be performed for a time e.g., for 1-24 hours, alternatively 1 to 8 hours. The amount of adsorbent may be 1% to 10%, alternatively 3% to 5%, based on combined weight of the metal oxide and the olefinic nitrile. Particle size of the metal oxide may be, for example, 1 mm to 2 mm. Without wishing to be bound by theory, it is thought that the metal in the metal oxide may form a complex of the metal and cyanide impurity, and the complex may then be removed by filtration.

Alternatively, the olefinic nitrile may be stripped or distilled, and/or sparged with nitrogen or another inert gas such as argon. The cyanide impurity may be removed in the initial "heads" cut, leaving the purified olefinic nitrile in the pot. This may be done near atmospheric pressure to limit the required temperature to achieve removal of the heads cut. When sparging with inert gas, the gas may be nitrogen.

Alternatively, two or more of the methods described above may be used, e.g., the olefinic nitrile may be contacted with the metal absorber and the adsorbent, either concurrently or sequentially (e.g., the olefinic nitrile may be contacted with the metal absorber, filtered, and thereafter contacted with the adsorbent such as activated carbon. Step 2) reduces the amount of cyanide impurity in A) the olefinic nitrile as compared to the amount of the cyanide impurity present in A') the olefinic nitrile provided in step 1). For example, the purified olefinic nitrile may contain 0 ppm of cyanide impurity. Alternatively, the purified organic nitrile may contain at least 10 ppm less of cyanide impurity after step 2) as compared to A') the olefinic nitrile provided in step 1).

Starting materials comprising A) the purified olefinic nitrile prepared as described above, B) a hydridosilane, and C) a platinum hydrosilylation reaction catalyst are combined in step 3) of the method described above. Combining may be performed by any convenient means such as mixing, optionally with heating. For example, step 3) may be performed at a temperature of 20°C to 150°C, preferably 60-100 °C, under inert atmosphere. Step 3) may be performed under an inert atmosphere. Pressure is not critical and may be, e.g., 0-345 kPa (0 to 50 psig); alternatively ambient pressure may be used for convenience. Step 3) may be performed batch-wise, semi-batch-wise or continuously, with heating or cooling to manage the reaction progress.

Starting material B), the hydridosilane, may have formula R¹ₓHSiX₍₃₋ₓ₎, where subscript x is 0, 1, or 2; each R¹ is an independently selected monovalent hydrocarbon group of 1 to 18 carbon atoms, and each X is independently selected from the group the group consisting of a halogen atom and an alkoxy group of formula OR¹. Alternatively, the hydridosilane may be an alkoxysilane. Examples of alkoxysilanes for starting material B) include methyldimethoxysilane, trimethoxysilane, ethyldimethoxysilane, triethoxysilane, ethyldiethoxysilane. Suitable hydridosilanes are known in the art and are commercially available, e.g., from Dow Silicones Corporation of Midland, MI, USA. Starting materials A) and B) may be used in amounts sufficient to provide 1:1 molar equivalents of A) and B). Alternatively, a molar excess of A) or B) may be used. For example, molar ratio of starting materials A) and B) (A/B ratio) may be 10/1 to 1/10; alternatively 2/1 to 1/2, alternatively 1/1.5 to 1.5/1, alternatively 1/1 to 1.1/1, and alternatively 1.1/1 to 1/1.

Starting material C) is the platinum hydrosilylation reaction catalyst. Platinum hydrosilylation reaction catalysts are known in the art and are commercially available. The platinum hydrosilylation reaction catalyst may comprise platinum metal, which may optionally be deposited on a carrier such as silica, alumina, or activated carbon. Alternatively, the platinum hydrosilylation reaction catalyst may be a compound of platinum, for example, chloroplatinic acid (Speier's Catalyst), chloroplatinic acid hexahydrate, or platinum dichloride. Alternatively, the platinum hydrosilylation reaction catalyst may be a complex of such a compound e.g., with low molecular weight organopolysiloxanes. Complexes of platinum with low molecular weight organopolysiloxanes include 1,3-diethenyl-1,1,3,3-tetramethyldisiloxane complexes with platinum (Karstedt's Catalyst). Alternatively, the platinum hydrosilylation reaction catalyst may be a platinum compound or a platinum complex, described above, microencapsulated in a matrix or coreshell type structure, e.g., said compound or complex may be microencapsulated in a resin matrix. Alternatively, the platinum hydrosilylation reaction catalyst may comprise chloroplatinic acid, Karstedt's catalyst, or platinum on a support selected from carbon, silica, or alumina. Exemplary platinum hydrosilylation reaction catalysts are described, for example, in U.S. Patent 2,823,218 to Speier; U.S. Patent 3,159,601 to Ashby; U.S. Patent 3,220,972 to Lamoreaux; U.S. Patent 3,419,593 to Willing; U.S. Patent 3,516,946 to Modic; U.S. Patent 3,814,730 to Karstedt; U.S. Patent 3,989,668 to Lee, et al.; U.S. Patent 4,766,176 to Lee, et al.; U.S. Patent 4,784,879 to Lee, et al.; U.S. Patent 5,017,654 to Togashi; U.S. Patent 5,036,117 to Chung, et al.; and U.S. Patent 5,175,325 to Brown, et al.; and EP 0 347 895 A to Togashi, et al. Platinum hydrosilylation reaction catalysts are commercially available, for example, SYL-OFF^{™} 4000 Catalyst and SYL-OFF^{™} 2700 are available from Dow Silicones Corporation of Midland, Michigan, USA. The platinum hydrosilylation reaction catalyst may be used in an amount sufficient to provide 5 ppm to 200 ppm, alternatively 5 ppm to < 200 ppm, alternatively 5 ppm to 100 ppm, and alternatively 35 ppm to 70 ppm, of platinum based on combined weights of starting materials A), B), and C).

The method described above may optionally further comprise adding starting material D), a promoter, during and/or after step 3). Hydrosilylation reaction promoters are known in the art, and can also be applied to improve reaction of olefinic nitriles.

Various carboxylic acids may be used as the promoter in the method described herein. For example, neodecanoic acid may be used as the promoter. When present, the carboxylic acid may be added in amount sufficient to provide > 0:1 to 1:1 carboxylic acid: (hydridosilane + olefinic nitrile), alternatively 0.005:1 to 0.02:1 for the following reasons: at a component ratio below 0.005: 1, the effects relative to product selectivity and product yield may become unsatisfactory; but ratios in excess of 0.02:1, while still accruing the advantageous effects of this invention, may result in excess material losses.

The method described above may optionally further comprise delivering one or more of the starting materials described above in E) a solvent. For example, starting material C) the hydrosilylation reaction catalyst, may be delivered in a solvent. Suitable solvents include organic liquids exemplified by, but not limited to, aromatic hydrocarbons, aliphatic hydrocarbons, alkyl halides and aromatic halides. Hydrocarbons include benzene, toluene, xylene, naphtha, hexane, cyclohexane, methylcyclohexane, heptane, octane, decane, hexadecane, isoparaffin such as Isopar L (C11-C13), Isopar H(C11-C12), hydrogenated polydecene. Alternatively, the solvent may be selected from polyalkylsiloxanes and aromatic hydrocarbons such as toluene, xylene, or a combination thereof. Polyalkylsiloxanes with suitable vapor pressures may be used as the solvent, and these include hexamethyldisiloxane, octamethyltrisiloxane, hexamethylcyclotrisiloxane, octamethylcyclotetrasiloxane, decamethylcyclopentasiloxane, tris(trimethylsiloxy)methylsilane, tetrakis(trimethylsiloxy)silane, dodecamethylcyclohexasiloxane, octamethyltrisiloxane, decamethyltetrasiloxane, dodecamethylpentasiloxane, tetradecamethylhexasiloxane, hexadecamethylheptasiloxane, heptamethyl-3-{(trimethylsilyl)oxy)}trisiloxane, hexamethyl-3,3, bis {(trimethylsilyl)oxy} trisiloxane pentamethyl{(trimethylsilyl)oxy } cyclotrisiloxane, and combinations thereof. Low molecular weight polyalkylsiloxanes, such as 0.5 to 1.5 cSt polydimethylsiloxanes are known in the art and commercially available as DOWSIL^{™} 200 Fluids and DOWSIL^{™} OS FLUIDS, which are commercially available from Dow Silicones Corporation.

The amount of solvent depends on various factors including the type and amount of each of starting materials A), B), and C), and, when present D), and their relative compatibilities with each other, and the type of equipment to be used to combine them. However, the amount of solvent may be 0 to 95% based on the weight of starting material C).

The method described above produces a product comprising a compound of formula R¹ₓR²SiX₍₃₋ₓ₎, where subscript x and R¹ are as defined above, and R² is a group of formula N≡C-D-, where D is a divalent hydrocarbon group (e.g., N≡C-CH(CH₃)-CH₂-CH₂-). The compound may be a cyanoalkyl-functional alkoxysilane (i.e., when X is OR¹). Cyanoalkyl-functional alkoxysilanes such as (3-cyanobutyl)methyldimethoxysilane are useful for treating electrical cables, as described for example, in U.S. Patent 8,656,586.

The product may further comprise an unreacted starting material, catalyst, promoter, and solvent, if used. Therefore, the method may optionally further comprise recovering the compound from the product. Recovering may be performed by any convenient means, such as stripping and/or distillation.

### EXAMPLES

These examples are intended to illustrate some embodiments of the invention and should not be interpreted as limiting the scope of the invention set forth in the claims. Starting materials used in these examples are described in Table 1.

**Table 1 - Starting Materials Used in the Examples**

| Starting Material | Chemical Description | Source |
|---|---|---|
| 2M3BN | 2-methyl-3-butenenitrile (CAS 16529-56-9) | Dytek 2M3BN, Invista |
| 3BN | 3-butenenitrile | Sigma Aldrich |
| MDMS | Methyldimethoxysilane (CH₃)HSi(OCH₃)₂ (CAS 16881-77-9) | Dow Silicones Corporation of Midland, Michigan, USA |
| | Neodecanoic acid | Brenntag Great Lakes, LLC |
| | Karstedt's catalyst diluted to 1 wt% Pt in either toluene or xylene solvent | Karstedt's catalyst from Dow Silicones Corporation was dissolved in xylene or toluene, both of which were from Univar Solutions USA INC |
| Cu | Copper powder, <425 µm, 99.5% trace metals basis | Sigma Aldrich |
| AC | Lignite based activated carbon Darco12x40 used in Example 2 | Norit |
| Ag | silver powder: spherical, average particle size 0.6 - 2 micron, purity 99.9% | Alfa Aesar |

In this Comparative Example 1, 2M3BN and methyldimethoxysilane were hydrosilylated under the following conditions. Reaction mixtures were prepared with 1.17 mL of methyldimethoxysilane, 1.19 mL of 2M3BN (a 25 % molar excess with respect to the methyldimethoxysilane), and 35 µL of neodecanoic acid in glass tube reactors, each of dimensions 19.7 cm (7.75") in length and 1.9 cm (¾") in diameter. Each tube reactor was chilled on dry ice, and then a solution of Karstedt's catalyst dissolved in toluene such that the resulting catalyst solution contained 1 weight % platinum was added targeting varying Pt concentrations in the reaction mixture in different tube reactors, as summarized below in Table 2. The cyanide impurity concentration in the 2M3BN was measured to be 45 ppm, which translated to a molar concentration of 140.4 mol cyanide impurity per 10⁶ mol 2M3BN. Hydrosilylation reactions were completed using different amounts of Pt catalyst (1 wt% Pt solution was made using toluene solvent to dilute Karstedt's catalyst, then varying amounts of 1 wt% Pt solution were added) at 100°C with a reaction time of 1 hour. The effluent was analyzed by GC, and conversion of the limiting reagent is shown in Table 2. This comparative example showed that conversion was significantly hindered as Pt concentration was reduced.

In this Example 2, the hydrosilylation of Comparative Example 1 was repeated, except that the 2M3BN was purified before hydrosilylation. The same 2M3BN was used in comparative example 1 was purified to reduce cyanide impurity content by passing through a bed of Cu followed by lignite-based Activated Carbon (AC). The mass ratio of Cu and AC to 2M3BN treated was 3%. The cyanide impurity concentration of the 2M3BN after treatment was measured to be 7.3 ppm, which translated to a molar concentration of 22.8 mol cyanide impurity per 10⁶ mol 2M3BN, an 83.8% reduction. Table 2 shows that high conversion of the limiting reagent was maintained as Pt concentration was reduced.

In this Example 3, the hydrosilylation of Example 2 was repeated, except the beds of Cu and AC were reused. The 2M3BN was the same as was used in Comparative Example 1. The purified 2M3BN used in this Example 3 was obtained as the fourth aliquot of material passed through the beds described in Example 2, each at a mass ratio of 3% Cu/AC to 2M3BN. Table 2 shows that conversion was maintained as Pt concentration was reduced. This Example 3 shows that the beds can be reused.

In this Comparative Example 4, the reaction of Comparative Example 1 was repeated, except 0.97 mL 3BN was used instead of 1.19 mL 2M3BN. The cyanide impurity concentration in the 3BN was measured to be 25.6 ppm, which translated to a molar concentration of 68 mol cyanide per 10⁶ mol 3BN.

In this Example 5, the reaction of Comparative Example 4 was repeated, except the 3BN was purified to reduce cyanide impurity content before use. Purification was performed by passing the 3BN through a bed of Cu and AC. The mass ratio of Cu and AC to 3BN treated was 3%. The cyanide impurity concentration after treatment was measured to be 5.2 ppm, which translates to a molar concentration of 13.8 mol cyanide per 10⁶ mol 3BN, a 79.6% reduction. This Example 5 showed that conversion was maintained even as Pt concentration was reduced.

**Table 2 - Comparative Examples 1 and 4 and Examples 2, 3 & 5**

| Pt concentration (ppm) | Limiting Reagent Conversion (%) | | | | |
|---|---|---|---|---|---|
| | Comparative Example 1 | Example 2 | Example 3 | Comparative Example 4 | Example 5 |
| 200 | 69.5 | 73.2 | 72.3 | 78.1 | 80.6 |
| 100 | 57.3 | 72.1 | 74.7 | 69.7 | 79.1 |
| 70 | 50.6 | 66.5 | 72.2 | 37.0 | 78.0 |
| 35 | 27.4 | 71.9 | 68.1 | 35.3 | 77.2 |

In this Reference Example 6, samples of 2M3BN were obtained and analyzed before and after purification to reduce cyanide impurity concentration. Metal treatment, when used, was performed simultaneously or before activated carbon treatment. When metal treatment was performed first, this is noted as 'stepwise' in Table 3, below. Metal treatment was performed by adding Cu powder or Ag powder (in amounts shown in Table 3) to native new 2M3BN. The samples were shaken vigorously for 1 day then filtered with a 0.45 µm syringe filter. When activated carbon treatment was performed, a milled or unmilled, acidic, basic, or neutral wood based activated carbon was added to the sample of filtered 2M3BN, and the sample was shaken for 1 day. The sample was then filtered through another 0.45 µm syringe filter. The CN- ion content was measured using an Ion Selective Electrode before and after treatment to determine the reduction in concentration. The samples are summarized below in Table 3.

**Table 3**

| 2M3BN Loading (g) | Starting CN-(ppm) | Metal | Metal Loading (g) | AC | AC Loading (g) | Treatment Order | CN-Remaining (ppm) | % CN Reduction | Cu (kps) | Approx Cu (ppm) |
|---|---|---|---|---|---|---|---|---|---|---|
| 8.2 | 72 | None | None | Wood | 0.355 | NA | 45 | 37 | | |
| 8.2 | 72 | None | None | Wood | 0.360 | NA | 52.5 | 27 | | |
| 8.2 | 61 | Cu | 0.065 | None | NA | NA | NA | NA | 155 | 300 |
| 4.1 | 58.5 | Ag | 0.026 | None | NA | NA | 23 | 59 | | |
| 8.2 | 72 | Cu | 0.064 | None | NA | NA | 17 | 77 | | |
| 8.2 | 47.5 | CuO | 0.064 | None | N/A | N/A | 28.5 | 40 | | |
| 8.2 | 61 | Cu | 0.063 | Milled Acidic | 0.304 | simultaneous | 5.2 | 91 | | |
| 8.2 | 61 | Cu | 0.065 | Acidic | 0.301 | simultaneous | 5.6 | 91 | 11.4 | 90 |
| 8.2 | 61 | Cu | 0.067 | Milled Basic | 0.359 | simultaneous | 15.5 | 75 | | |
| 8.2 | 61 | Cu | 0.062 | Basic | 0.303 | simultaneous | 19.5 | 68 | | |
| 8.2 | 61 | Cu | 0.072 | Milled Neutral | 0.321 | simultaneous | 26 | 57 | | |
| 8.2 | 61 | Cu | 0.065 | Neutral | 0.330 | simultaneous | 54.5 | 11 | | |
| 8.2 | 61 | Ag | 0.061 | Milled Acidic | 0.312 | simultaneous | 38.5 | 37 | | |
| 8.2 | 61 | Ag | 0.072 | Acidic | 0.365 | simultaneous | 40.5 | 33 | | |
| 8.2 | 61 | Cu | 0.065 | Wood Acid | 0.35 | stepwise | NA | NA | 3.1 | 6 |
| 8.2 | 61 | Cu | 0.065 | Wood Base | 0.35 | stepwise | NA | NA | 5.1 | 10 |
| 8.2 | 61 | Cu | 0.065 | Wood Neutral | 0.35 | stepwise | NA | NA | 5.8 | 11 |
| 28.7 | 150 | Cu | 0.247 | Wood Acid | 1.115 | stepwise | 20.5 | 86 | | |

In this Reference Example 7, the purified 2M3BN prepared according to the last line of Table 3, above, and comparative unpurified 2M3BN were used in the following hydrosilylation reaction. Tube headspace of nitrogen was used for these experiments. 2.5 mL of methyldimethoxysilane, 2.5 mL of the purified 2M3BN, and 44 µL of neodecanoic acid were added in glass tube reactors, each of dimensions 19.7 cm (7.75") in length and 1.9 cm (¾") in diameter. Each tube reactor was chilled on dry ice, and then 20 µL of a catalyst solution was added targeting 50 ppm Pt in the reaction mixture, followed by placing the tube on a heater block at 80°C. The catalyst solution was Karstedt's catalyst diluted in xylene, such that the resulting catalyst solution contained 1 weight % Pt. Tubes were left on the heater block for 1 hour and then GC was used to measure reaction progression. The results shown below in Table 4 showed that stepwise treatment provided a beneficial result under the conditions tested.

**Table 4**

| | Weight% | | | | | Reaction Metrics | |
|---|---|---|---|---|---|---|---|
| 2M3BN | MDM S | 2M3B N | 2M3B N Isomers | Product | High boiler s | Conv. MDM S | Yield |
| Comparative Sample 7-1 using 2M3BN treated without purification first | 33.0% | 32.8% | 5.8% | 27.1% | 1.2% | 33.0% | 31.2% |
| Sample 7 - 2 Using 2M3BN treated with Cu then AC stepwise, see last line of Table 3 | 22.8% | 25.4% | 6.2% | 42.4% | 3.1% | 53.6% | 48.7% |

In this Reference Example 8, 2M3BN was purified using various adsorbents followed by use in a hydrosilylation reaction. Purification was performed as follows: Adsorbents were added to 5 mL of 2M3BN so that liquid level was just above the height of the adsorbent. The resulting mixtures were soaked for 5 days with periodic shaking. Thereafter, the liquid was syringe filtered from the adsorbent using a 0.2 µm filter. Table 5 summarizes the 2M3BN samples prepared.

**Table 5 - Adsorbents Used for Purification of 2M3BN**

| Example | Name | Vendor | Functionality | Base | Comments |
|---|---|---|---|---|---|
| 8a | Control | None | none | none | Comparative Example, control with no treatment of the 2M3BN |
| 8b | 3A molecular sieves, 8 to 12 mesh | Sigma Aldrich | None | none | Sigma Aldrich |
| 8c | Pricat CZ 40/18 T | Johnson Matthey | CuO/ZnO/AlO | N/A | Metal oxides were used instead of metal powders. |
| 8d | DARCO 12 x20 | Cabot | Activated Carbon | Lignite Coal | water white 2M3BN was obtained |
| 8e | CE 12x30 | Calgon | 0.5% Ag/C | Coconut shells | Ag metal on activated carbon support |

The 2M3BN samples in Table 5 were then used in a hydrosilylation reaction, as follows: Tube headspace of nitrogen was used for these experiments. 1.75 mL of methyldimethoxysilane, 1.35 mL of 2M3BN, and 28 µL of neodecanoic acid were added to a glass tube of the same dimensions as in Comparative Example 1. Each tube was chilled on dry ice, and then 75 µL of catalyst solution was added targeting 250 ppm Pt in the reaction mixture, followed by placing the tube on a heater block at 80°C. The catalyst solution was of Karstedt's catalyst diluted in xylene, such that the solution contained 1% Pt. After 5 hours, GC analysis was performed to measure reaction progression. Results are shown below in Table 6.

**Table 6 - Experimental Reaction Results (80°C, 250 ppm Pt, SiH:2M3BN=1.2, 1% acid, 5 hours)**

| Weight % | | | | | | Reaction Metrics | | | Result |
|---|---|---|---|---|---|---|---|---|---|
| Example | MDMS | 2M3BN | 2M3BN Isomers | Product | High boilers | Conv. 2M3BN | Conv. MDMS | Yield | |
| 8a | 35.3% | 28.4% | 11.1% | 23.9% | 1.4% | 26.7% | 27.7% | 26.52% | Comparative Example |
| 8b | 32.3% | 23.7% | 10.6% | 31.8% | 1.6% | 36.7% | 35.8% | 35.33% | 33% improvement in Yield |
| 8c | 48.4% | 0.0% | 50.7% | 0.1% | 0.7% | NA | NA | 0.15% | Negative result for use of metal oxides, which inhibited reaction |
| 8d | 28.6% | 24.1% | 10.6% | 34.5% | 2.1% | 38.3% | 40.6% | 38.34% | 44% Yield Improvement |
| 8e | 27.1% | 20.8% | 10.1% | 39.8% | 2.2% | 45.3% | 45.4% | 44.23% | 67% improvement in Yield |

This Reference Example 8 showed that purification of 2M3BN with silver provided an improvement in hydrosilylation synthesis yields. Treatment of 2M3BN with activated carbon and molecular sieves resulted in modest improvement of hydrosilylation yields. Without wishing to be bound by theory, it is thought that the negative result in 8c was due to inhibition of the reaction by the metal oxide and/or the complex of metal oxide with the cyanide impurity. Therefore, it is further thought that when metal oxides are used to remove the cyanide impurity, any metal oxide and/or complex thereof should be removed, e.g., via filtration before step 3) of the method described herein.

In this Example 9, cyanide impurity was removed from 2M3BN via batch distillation followed by hydrosilylation. A batch distillation was performed using a 1L 3 neck flask and 10 stage trayed glass column. A water condenser was used to condense material and collect it in a 50 mL receiver. A heating mantle was used to provide heat input to the flask and a stirrer bar provided mixing. 450 grams of 2M3BN was loaded to the flask. The flask was heated and 30.1 grams of material was distilled overhead at a 1:1 reflux ratio. The purified 2M3BN remaining in the flask was found to be reduced in cyanide impurity from 53 to 8 µg/mL.

Both the starting 2M3BN and the purified 2M3BN were used in hydrosilylation reactions for comparison. The hydrosilylation reaction was performed in a semi-batch manner. 270g of 2M3BN, 13.6g of neodecanoic acid, and 0.42g of Karstedt's catalyst (24% Pt) were loaded to a 3-neck flask. 333.7g of methyldimethoxysilane was loaded to a syringe pump. 30 g of 2M3BN was mixed with 13.6g of neodecanoic acid and added to a second syringe pump. The contents of the syringe pumps were metered in over a period of 6 hours. Liquid temperature of the reactor contents was maintained between 80-85 °C. GC was used to measure the extent of reaction.

In comparative sample 9-1, the reaction performed with control 2M3BN containing 53 µg/mL cyanide impurity resulted in 97.98% conversion of methyldimethoxysilane. The reaction performed with the purified 2M3BN containing 8 µg/mL cyanide impurity resulted in 99.99% conversion of methyldimethoxysilane.

Purifying 2M3BN by distillation was effective at reducing cyanide impurity concentration. The purified 2M3BN had improved hydrosilylation reaction performance, reducing residual SiH by 99.5% more than the control. The samples had 96 ppm residual SiH in reaction crude when control 2M3BN was used as compared to only 0.4 ppm SiH in the reaction crude when the purified 2M3BN was used. Achieving low levels of SiH is important for safe disposal of the waste.

In this Example 10, distillation of 2M3BN to reduce cyanide impurity content and give improved hydrosilylation performance was performed as follows. A batch distillation with a 250 mL 3 neck flask and 10 stage trayed glass column was used. A water condenser was used to condense material and collect it in a 50 mL receiver. A heating mantle was used to provide heat input to the 3 neck flask and a stirrer bar provided mixing. 148.8 g of TCI sourced 2M3BN was loaded to the flask. The flask was heated to 122 °C and the column was put on total reflux for 1 hour. Then various overheads cuts were collected.

Hydrosilylation reactions were performed with the various distillation cuts. Tube headspace of nitrogen was used for these experiments. 1.0 mL of methyldimethoxysilane, 0.8 mL 2M3BN were added to glass tubes of the dimensions in Comparative Example 1. The tubes were chilled on dry ice and then catalyst solution was added targeting 100 ppm Pt in the reaction mixture. Tubes were placed on a heater block at 100°C for 2 hours and then GC was used to measure reaction progression. The results are shown below in Table 7.

**Table 7 - Experimental Reaction Results (100 °C, 100 ppm Pt, SiH:2M3BN=1, 2 hours)**

| | Experimental Conditions | | Reaction Weight % | | | | | Reaction Metrics | | |
|---|---|---|---|---|---|---|---|---|---|---|
| E x 9 | Cut | Cut Amou nt (g) | MDM S | 2M3B N | 2M3B N Isomer s | Produ ct | High boiler s | Conv. 2M3B N | Conv. MDM S | Yield |
| a | Compa r-ative | | 41.6% | 42.1% | 12.1% | 4.0% | 0.1% | 4.0% | 5.2% | 4.03% |
| b | 1 ^{st} cut | 5.6 | 38.1% | 45.5% | 15.8% | 0.5% | 0.0% | 0.5% | 0.7% | 0.50% |
| c | 2^{nd} cut | 14.8 | 38.3% | 47.4% | 13.3% | 1.0% | 0.0% | 0.9% | 1.5% | 1.02% |
| d | 3^{rd} cut | 8.2 | 38.3% | 47.6% | 12.6% | 1.4% | 0.0% | 1.3% | 2.1% | 1.43% |
| e | 4^{th} cut | 10.6 | 30.2% | 48.1% | 13.1% | 8.5% | 0.1% | 7.1% | 13.8% | 8.52% |
| f | 5^{th} cut | 18.8 | 24.1% | 39.0% | 12.1% | 23.7% | 1.1% | 20.9% | 35.8% | 23.69 % |
| g | 6^{th} cut | 42.6 | 23.2% | 44.6% | 12.2% | 19.2% | 0.8% | 15.7% | 32.0% | 19.21 % |
| h | 7^{th} cut | 17.6 | 27.7% | 43.0% | 11.5% | 17.0% | 0.8% | 14.6% | 25.8% | 16.95 % |
| | 8^{th} cut | 12.3 | 30.8% | 43.8% | 11.4% | 13.4% | 0.6% | 11.7% | 19.8% | 13.37 % |

Without wishing to be bound by theory, it is thought that this Example 9 showed that 2M3BN can be distilled to improve hydrosilylation reactivity. Cuts from early in the distillation which contained higher amounts of the (lower-boiling) cyanide impurity performed very poorly in hydrosilylation reaction, even more poorly than the control. Meanwhile, 2M3BN cuts from later in the distillation, e.g., Cuts 4-8) contained less cyanide impurity and performed much better than the control (using undistilled 2M3BN) in hydrosilylation reactions.

### Industrial Applicability

The inventors surprisingly found that cyanide impurity can act as an inhibitor to hydrosilylation of an olefinic nitrile and a hydridosilane. Specifically, cyanide impurity content was found to be directly related to loss of activity of platinum catalyst. Reducing the amount of cyanide impurity in the olefinic nitrile by as little as 10 ppm was found to increase the conversion of the limiting reagent at the same or lower platinum metal content.

### Usage of Terms

Abbreviations used in this application are as defined below in Table 8.

**Table 8**

| Abbreviation | Definition |
|---|---|
| °C | degrees Celsius |
| cm | centimeters |
| g | grams |
| GC | Gas chromatography (according to the test method described below) |
| L | liters |
| 2M3BN | 2-methyl-3-butenenitrile (CAS 16529-56-9) |
| MDMS | Methyldimethoxysilane (CAS 16881-77-9) |
| mM | meters |
| mL | milliliters |
| mol | mole |
| ppm | parts per million |
| µ | micro |
| µL | microliters |
| µm | micrometers |
| wt | weight |

Samples were analyzed by GC under the following conditions:
The gas chromatograph used was an Agilent 7890A running Chemstation OpenLab CDS Revision C.01.07 using an RTX-1 column (30m x 250µm x 1.00µm nominal) on a flame ionization detector (FID). An autosampler was used for sample injection. The method parameters were as follows:
- Start: 50 °C - 1 minute hold
- Ramp: 15°C/minute
- End: 260°C - 5 minute hold
- Injection Port: 250 °C
- Detector: 300 °C
- Helium flow - 2.1 mL/minute
- Split 50:1
- Injection Volume: 1.0 µL

## Claims

1. A method comprising:
1) providing A') an olefinic nitrile comprising a cyanide impurity of formula X'-C≡N, where X' is hydrogen, sodium, or potassium,
2) removing all or a portion of the cyanide impurity from A') the olefinic nitrile, thereby producing A) a purified olefinic nitrile; wherein step 2) comprises one or more of
combining A') the olefinic nitrile and an elemental metal selected from the group consisting of copper, iron, silver, or nickel; or
combining A') the olefinic nitrile and an adsorbent selected from the group consisting of activated carbon and molecular sieves; or
combining A') the olefinic nitrile to a metal selected from the group consisting of copper, iron, silver, or nickel, or the oxides of these metal; and thereafter combining the product and an absorbent to form A) the purified olefinic nitrile; and
3) combining, at a temperature of 20 °C to 150 °C, starting materials comprising
A) the purified olefinic nitrile;
B) the hydridosilane, where the hydridosilane has formula R1ₓHSiX₍₃₋ₓ₎, where subscript x is 0, 1, or 2; each R¹ is an independently selected monovalent hydrocarbon group of 1 to 18 carbon atoms, and each X is independently selected from the group consisting of a halogen atom and an alkoxy group of formula OR¹; and
C) a platinum hydrosilylation reaction catalyst in an amount sufficient to provide 5 ppm to < 200 ppm Pt based on combined weights of starting materials A), B), and C).

2. The method of claim 1, where A') the olefinic nitrile is selected from the group consisting of 3-butenenitrile and 2-methyl-3-butenenitrile.

3. The method of claim 1 or claim 2, where in A'), the cyanide impurity comprises 25 ppm to 125 ppm of H-C≡N.

4. The method of any one of claims 1 to 3, where B' the hydridosilane has x is 0 or 1, R¹ is an alkyl group, and X is an alkoxy group.

5. The method of claim 4, where x = 1, R¹ is a methyl group, and each X is a methoxy group.

6. The method of any one of claims 1 to 5, where the platinum hydrosilylation reaction catalyst comprises chloroplatinic acid, Karstedt's catalyst, or platinum on a support selected from carbon, silica, or alumina.

7. The method of any one of the preceding claims, where step 2) comprises stripping, distillation, or a combination thereof.

8. The method of any one of the preceding claims, where A) the purified olefinic nitrile produced in step 2) contains 0 ppm of cyanide impurity to at least 10 ppm less of cyanide impurity than A') the olefinic nitrile provided in step 1).

9. The method of claim 8, where A) the purified olefinic nitrile contains 5 ppm to 40 ppm of cyanide impurity.

10. The method of any one of the preceding claims, further comprising: adding D) a promoter during and/or after step 3).

11. The method of any one of the preceding claims, further comprising delivering one or more starting materials in a solvent.

12. The method of any one of the preceding claims, where the method produces a product comprising a compound of formula R¹ₓR²SiX₍₃₋ₓ₎, where subscript x and R¹ are as defined above, and R² is a group of formula N≡C-D-, where D is a divalent hydrocarbon group.

## Patentansprüche

1. Verfahren, umfassend:
1) Bereitstellen von A') einem olefinischen Nitrils, umfassend eine Cyanidverunreinigung der Formel X'-C=N, wobei X' Wasserstoff, Natrium oder Kalium ist,
2) Entfernen der gesamten oder eines Teils der Cyanidverunreinigung aus A') dem olefinischen Nitril, wodurch A) ein gereinigtes olefinisches Nitril produziert wird; wobei Schritt 2) eines oder mehrere umfasst von
Kombinieren von A') dem olefinischen Nitril und einem elementaren Metall, ausgewählt aus der Gruppe bestehend aus Kupfer, Eisen, Silber oder Nickel; oder
Kombinieren von A') dem olefinischen Nitril und einem Adsorptionsmittel, ausgewählt aus der Gruppe, bestehend aus Aktivkohle und Molekularsieben; oder
Kombinieren A') des olefinischen Nitrils mit einem Metall, ausgewählt aus der Gruppe, bestehend aus Kupfer, Eisen, Silber oder Nickel oder den Oxiden dieser Metalle; und danach Kombinieren des Produkts und eines Absorptionsmittels, um A) das gereinigte olefinische Nitril auszubilden; und
3) Kombinieren, bei einer Temperatur von 20 °C bis 150 °C, von Ausgangsmaterial, umfassend
A) das gereinigte olefinische Nitril;
B) das Hydridosilan, wobei das Hydridosilan die Formel R¹_{X}HSiX₍₃₋ₓ₎ aufweist, wobei der tiefgestellte Index x 0, 1 oder 2 ist; jedes R¹ eine unabhängig ausgewählte einwertige Kohlenwasserstoffgruppe mit 1 bis 18 Kohlenstoffatomen ist, und jedes X unabhängig aus der Gruppe ausgewählt ist, bestehend aus einem Halogenatom und einer Alkoxygruppe der Formel OR¹; und
C) einen Platinhydrosilylierungsreaktionskatalysator in einer Menge, die ausreicht, um 5 ppm bis < 200 ppm Pt, bezogen auf kombinierte Gewichtungen der Ausgangsmaterialien A), B) und C), bereitzustellen.

2. Verfahren nach Anspruch 1, wobei A') das olefinische Nitril aus der Gruppe ausgewählt ist, bestehend aus 3-Butennitril und 2-Methyl-3-butennitril.

3. Verfahren nach Anspruch 1 oder 2, wobei in A') die Cyanidverunreinigung 25 ppm bis 125 ppm H-C=N umfasst.

4. Verfahren nach einem der Ansprüche 1 bis 3, wobei B' das Hydridosilan aufweist x 0 oder 1 ist, R¹ eine Alkylgruppe ist und X eine Alkoxygruppe ist.

5. Verfahren nach Anspruch 4, wobei x = 1, R¹ eine Methylgruppe ist und jedes X eine Methoxygruppe ist.

6. Verfahren nach einem der Ansprüche 1 bis 5, wobei der
Platinhydrosilylierungsreaktionskatalysator Chlorplatinsäure, Karstedt-Katalysator oder Platin auf einem Träger, ausgewählt aus Kohlenstoff, Siliciumdioxid oder Aluminiumoxid, umfasst.

7. Verfahren nach einem der vorstehenden Ansprüche, wobei Schritt 2) Abscheiden, Destillation oder eine Kombination davon umfasst.

8. Verfahren nach einem der vorstehenden Ansprüche, wobei A) das gereinigte olefinische Nitril, das in Schritt 2) produziert wird, 0 ppm Cyanidverunreinigung bis mindestens 10 ppm weniger Cyanidverunreinigung enthält als A') das olefinische Nitril, das in Schritt 1) bereitgestellt wird.

9. Verfahren nach Anspruch 8, wobei A) das gereinigte olefinische Nitril 5 ppm bis 40 ppm Cyanidverunreinigungen enthält.

10. Verfahren nach einem der vorstehenden Ansprüche, ferner umfassend: Hinzufügen D) eines Promotors während und/oder nach Schritt 3).

11. Verfahren nach einem der vorstehenden Ansprüche, ferner umfassend ein Abgeben eines oder mehrerer Ausgangsmaterialien in einem Lösungsmittel.

12. Verfahren nach einem der vorstehenden Ansprüche, wobei das Verfahren ein Produkt produziert, umfassend eine Verbindung der Formel R¹ₓR²SiX₍₃₋ₓ₎,
wobei der tiefgestellte Index x und R¹ wie oben definiert sind, und R² eine Gruppe der Formel N=C-D- ist, wobei D eine zweiwertige Kohlenwasserstoffgruppe ist.

## Revendications

1. Procédé comprenant :
1) la fourniture de A') un nitrile oléfinique comprenant une impureté de cyanure de formule X'-C=N, où X' est hydrogène, sodium, ou potassium,
2) l'élimination de tout ou partie de l'impureté de cyanure de A') le nitrile oléfinique, permettant ainsi de produire A) un nitrile oléfinique purifié ; dans lequel l'étape 2) comprend une ou plusieurs parmi
la combinaison de A') le nitrile oléfinique et un métal élémentaire choisi dans le groupe constitué de cuivre, fer, argent, ou nickel ; ou
la combinaison de A') le nitrile oléfinique et un adsorbant choisi dans le groupe constitué de charbon actif et tamis moléculaires ; ou
la combinaison de A') le nitrile oléfinique à un métal choisi dans le groupe constitué de cuivre, fer, argent, ou nickel, ou les oxydes de ces métaux ; puis la combinaison du produit et d'un absorbant pour former A) le nitrile oléfinique purifié ; et
3) la combinaison, à une température de 20 °C à 150 °C, de matières de départ comprenant
A) le nitrile oléfinique purifié ;
B) l'hydridosilane, où l'hydridosilane a la formule R¹ₓHSiX₍₃₋ₓ₎, où l'indice x vaut 0, 1, ou 2 ; chaque R¹ est un groupe hydrocarboné monovalent de 1 à 18 atomes de carbone, choisi indépendamment, et chaque X est choisi indépendamment dans le groupe constitué d'un atome d'halogène et d'un groupe alcoxy de formule OR¹ ; et
C) un catalyseur de réaction d'hydrosilylation au platine en une quantité suffisante pour fournir de 5 ppm à < 200 ppm de Pt sur la base des poids combinés des matières de départ A), B), et C) ;

2. Procédé selon la revendication 1, où A') le nitrile oléfinique est choisi dans le groupe constitué de 3-butènenitrile et 2-méthyl-3-butènenitrile.

3. Procédé selon la revendication 1 ou la revendication 2, où, dans A'), l'impureté de cyanure comprend de 25 ppm à 125 ppm de H-C=N.

4. Procédé selon l'une quelconque des revendications 1 à 3, où, dans B' l'hydridosilane, x vaut 0 ou 1, R¹ est un groupe alkyle, et X est un groupe alcoxy.

5. Procédé selon la revendication 4, où x = 1, R¹ est un groupe méthyle, et chaque X est un groupe méthoxy.

6. Procédé selon l'une quelconque des revendications 1 à 5, où le catalyseur de réaction d'hydrosilylation au platine comprend de l'acide chloroplatinique, un catalyseur de Karstedt, ou du platine sur un support choisi parmi carbone, silice, ou alumine.

7. Procédé selon l'une quelconque des revendications précédentes, où l'étape 2) comprend une extraction, une distillation, ou une combinaison de celles-ci.

8. Procédé selon l'une quelconque des revendications précédentes, où A) le nitrile oléfinique purifié produit à l'étape 2) contient de 0 ppm d'impureté de cyanure à au moins 10 ppm de moins d'impureté de cyanure que A') le nitrile oléfinique fourni à l'étape 1).

9. Procédé selon la revendication 8, où A) le nitrile oléfinique purifié contient de 5 ppm à 40 ppm d'impureté de cyanure.

10. Procédé selon l'une quelconque des revendications précédentes, comprenant en outre l'ajout de D) un promoteur pendant et/ou après l'étape 3).

11. Procédé selon l'une quelconque des revendications précédentes, comprenant en outre la distribution d'une ou plusieurs matières de départ dans un solvant.

12. Procédé selon l'une quelconque des revendications précédentes, où le procédé produit un produit comprenant un composé de formule R¹ₓR²SiX₍₃₋ₓ₎, où l'indice x et R¹ sont tels que définis ci-dessus, et R² est un groupe de formule N=C-D-, où D est un groupe hydrocarboné divalent.
